# EUROPEAN PATENT APPLICATION

(11) **EP 1 953 133 A1**
(43) Date of publication of application: **06.08.2008**
(21) Application number: 07001791.8
(22) Date of filing: 26.01.2007
(51) Int. Cl.: C07C 37/01, C07C 39/11, B01J 3/04

(54) **Process and apparatus for the production of hydroxytyrosol from olive oil extraction residues**

(71) Applicant: Probelte Pharma, S.A., 30100 Espinardo Murcia (ES)
(72) Inventor: Lopez Mas, José A., 30840 Alhama de Murcia Murcia (ES); Streitenberger, Sergio A., 30120 El Palmar Murcia (ES); Penalver Mellado, Marcos, 30007 Murcia Murcia (ES); Martinez Ortiz, Pedro, 30150 La Alberca Murcia (ES)
(74) Representative: Gislon, Gabriele

(57) **Abstract**

Hydroxytyrosol is extracted from a by-product of the olive oil extraction, by carrying out acid hydrolysis of said by-product at a temperature within the range of 105°C to 140°C and at a pH within the range of 1.0 to 6.0, at a pressure of 10 to 20 psi.

## Description

Process and apparatus for the production of hydroxytyrosol from olive oil extraction residues.

The present invention relates to a process and an apparatus for the production of hydroxytyrosol from the residues of the extraction of olive oil. More particularly, the invention relates to the production of hydroxytyrosol-containing products to be used as a source of hydroxytyrosol in food, medical and cosmetic industries.

It is now well known that the residues, i.e. the by-products, of the extraction of olive oil from olives contain a number of bioactive compounds, particularly polyphenols; among these polyphenols, hydroxytyrosol is of outstanding biological significance in view of its antioxidant, antimicrobial and radical scavenging activity.

Hydroxytyrosol is present in olive oil and in the residues of the olive oil industry; hydroxytyrosol has the following formula:

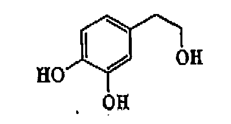

Production of hydroxytyrosol from extraction residues was and is actively investigated; an efficient extraction process could be very profitable.

It is known to obtain hydroxytyrosol from different residues of olive oil extraction. The residues can be classified in:
- vegetation water (in spanish: alpechin), i.e. the water that separates from oil as obtained by the pressing of olives without additional steps (classical olive oil extraction) or as obtained by the so-called three phases process in which water is added to chopped olives and the mix is centrifugated;
- pomaces, i.e. the solid residues of the pressing (in spanish: orujo), or of the three-phases process (orujo), or of the two-phases process, in which no water is added to the chopped olives in the centrifugation step (in spanish: alperujo).

For the purposes of the present description the term "pomaces" or "solid residue" is designating both "orujo" and "alperujo".

It is known to carry out acid hydrolysis of the pomaces (or vegetation water) to have the cleavage of the ester bond in the oleoeuropeine molecule and obtain hydroxytyrosol.

WO 2004/005228 discloses hydrolysis at room temperature of vegetation water obtained from olive oil extraction. This process requires the incubation of the acidified vegetation water until at least the 50% (preferably 90%) of the oleuropein originally present in the vegetation water has been converted to hydroxytyrosol. This step lasts for at least two months and even more, for example for approximately 6-12 months. Furthermore, the incubated vegetation water is extracted with an organic solvent, for example ethyl acetate, or it is contacted with a supercritical fluid (CO₂), to produce a rich fraction in hydroxytyrosol. The main problem connected with this process is the very long time required for the incubation of the acidified vegetation water, thus rendering this process non convenient from an economical and industrial point of view. In addition, the use of organic solvents should, if possible, be avoided, particularly when the final product obtained is to be used in the alimentary, cosmetic and pharmaceutical field.

US-B-6361803 discloses hydrolysis of olive pulp (pomaces) residues after oil extraction in acidic conditions at reflux for one hour (ex.12). Extraction temperature is said to be within the range of 20°C to reflux temperature. The extracted water solution is then loaded on a XAD-7 column and the column was washed with methanol. US-B-6361803 requires the use of organic solvents, particularly polar solvents, in order to recover hydroxytyrosol with an acceptable purity grade. Polar aqueous solvents are selected among methanol, ethanol, acetonitrile or acetone, while polar organic solvents are selected, for example, among esters, amides, dimethyl sulfoxide, dioxane, DMF and their mixtures. Most of these solvents are toxic and very difficult to completely eliminate from the desired hydroxytyrosol product. Accordingly, traces of the utilized solvents will be found in the final product even after several purification steps, thus rendering the hydroxytyrosol obtained according to this process not suitable for a safe application in the alimentary, cosmetic and pharmaceutical field.

US 2004/0102657 discloses acidic hydrolysis with a steam explosion process. The obtained solution is firstly partially purified on a column with non activated ion exchange resin and subsequently loaded on a XAD non-ionic column, where the column is washed with methanol or ethanol. The steam explosion process is a very severe treatment where the starting material is subjected to high temperatures (about 190-220°C). This step generates several by-products that are difficult to remove and, in addition, by-products formation is responsible for a poor yield in hydroxytyrosol, considering the process in its entirety: As by-products must be eliminated to purify hydroxytyrosol, the use of organic solvents is required in order to purify the hydroxytyrosol during the subsequent purification steps. The use of organic solvents, for example methanol that is a toxic solvent, is inconvenient, particularly when the final product obtained is to be used in the alimentary, cosmetic and pharmaceutical field.

Summarizing, the above mentioned techniques are either too long or too complex, or too harsh, or all of the above; this results in that the amount of remaining oleuropein, i.e. the amount that is not hydrolyzed, and/or the amount of the hydrolysis by-products is high enough to impart a bitter taste and a colour of the extract containing hydroxytyrosol and to therefore to make difficult the subsequent purification steps.

It is an aim of the present invention to solve the above mentioned problems and to provide a process of producing hydroxytyrosol from olive oil extraction by-products that is simple, effective, not expensive and that gives a high purity product.

Such aim is achieved by means of the present invention that provides a process according to claim 1. This process entails the acidic hydrolysis in water of the starting materials at a temperature above the reflux temperature and preferably in an autoclave. The pH of the acidified mixture that undergoes the hydrolysis is within the range of 1.0 to 6.0. The mixture after the hydrolysis is clarified by physical methods known in the art, e.g. by filtering and/or centrifuging, to remove the suspended solids from the hydrolysed product, and to obtain a clarified solution substantially free of solids in suspension.

According to a preferred embodiment of the invention the above steps are followed by the steps of loading the product thus obtained in at least one chromatographic column of a resin selected from acid activated anion exchange resins, and adsorbent non-ionic resins and of eluting the products retained in said chromatographic columns with water.

According to a further aspect of the invention the liquid product is concentrated by reverse osmosis concentration. According to a further step, after chromatographic purification and reverse osmosis concentration, the resulting liquid product is brought to a solid form, e.g. by freeze-drying, vacuum rotaevaporation or spray drying, with or without carriers such as maltodextrines.

A further object of the inventions are the hydroxytyrosol containing products as obtainable according to the above mentioned process. These products may be either in liquid or solid form and are characterized by having a hydroxytyrosol content of at least 0.5% (w/w) and a purity of at least 40% and preferably of at least 80% and more preferably of at least 95% (as determined by HPLC peak area measured at 280nm).

According to a preferred aspect of the invention, the hydroxytyrosol containing solid product obtainable according to the invention, has a hydroxytyrosol content of at least 90% (w/w), a purity of at least 90% (by HPLC 280nm) and a total phenols content of at least 92%.

A further object of the invention is an apparatus for carrying out the process as above discussed, characterized according to claim 13.

According to a preferred aspect of the invention, the apparatus, or plant, comprises at least one chromatographic column containing a resin selected from acid activated anion exchange resin that is a weakly basic, water elutable anion exchange resin and an adsorbent non-ionic resin that is a macroreticular cross-linked aromatic polymer resin.

The invention provides several advantages over the prior art techniques.

First of all, the hydrolysis step is carried out, according to the present invention, using just water and mineral acid, keeping the temperature above the reflux temperature using a combination of heating and pressure. When pressure is applied together with the claimed heating temperature, the resulting hydrolysis reaction is almost completed in about half an our, without significant formation of by-products and with a very good conversion of the starting material (oleuropein) into the final desired product (hydroxytyrosol). The hydrolysis step according to the present invention allows to obtain, in a short time, a very good yield in hydroxytyrosol together with a quite complete absence of those by-products, that are difficult to eliminate and which are instead formed, according to the prior art, in significant amounts.

The hydrolysis step, carried out according to the invention, provides for another advantage. The combined use of acids and temperature, beside its main objective, that is to carry out the hydrolysis with a good conversion rate and avoiding the formation of detrimental by-products, results in a sterilization of the mixture. In fact, the hydrolysis step provides a sterilization of the water solution, i.e. of the products involved, that is very useful, as the starting material (for example pomaces or olive waste) used in the processes for the preparation of hydroxytyrosol, usually comes from already treated materials, and generally requires some depuration-sterilization pre-treatments, to give a safe hydroxytyrosol final product. Said depuration-sterilization pre-treatments are often complex treatments and require additional process steps that, at the end of the full process, result in a lower yield in the desired final product. According to the present invention, the hydrolysis step contemporaneously allows to carry out the hydrolysis reaction and provides for the necessary sterilization of the products involved, thus avoiding the necessity to carry out any sterilization step on the starting material as well as providing for a hydrolysed material that is ready for further uses, without any additional sterilization treatment.

In addition, the hydrolysis step is preferably followed by at least a purification step, which is carried out by loading the product obtained from the hydrolysis step in at least one chromatographic column and eluting the hydroxytyrosol retained in said chromatographic column with water. In this case, as there are very low amounts of by-products coming from the hydrolysis step, it is possible to make use of a chromatographic column that can be eluted with water in the absence of any organic solvent, either alone or mixed with water. This means that no organic solvents are contacted with the hydroxytyrosol, thus obtaining a purified hydroxytyrosol that is particularly suitable to be used in the alimentary, cosmetic and pharmaceutical field.

Another advantage of the present invention is due to the concentration step, that allows to enrich the hydroxytyrosol content either after the hydrolysis and solid separation steps or after the purification steps. This concentration step provides a "concentrate" characterized by a high content of hydroxytyrosol, which can be purified or, if already purified, can directly be processed for further treatments. According to the prior art, where no "concentration" steps are provided in the described processes for hydroxytyrosol preparation, the hydroxytyrosol concentration in the processed solutions/dispersions is always very low, thus resulting in the necessity to operate with great volumes with consequent lower yields in the in the final product.

Always according to the present invention, the possibility to obtain a solid final product that is not mixed with any carrier, for example maltodextrines, gives the opportunity to formulate purified hydroxytyrosol according to its final intended use and according to any formal requirement, possibly required.

The invention will now be further disclosed in greater detail with reference to the enclosed non-limiting drawings wherein:
- fig.1 is a schematic view of an apparatus according to the invention; and
- figures 2-4 are chromatograms of three hydroxytyrosol containing products according to three embodiments of the invention process. :

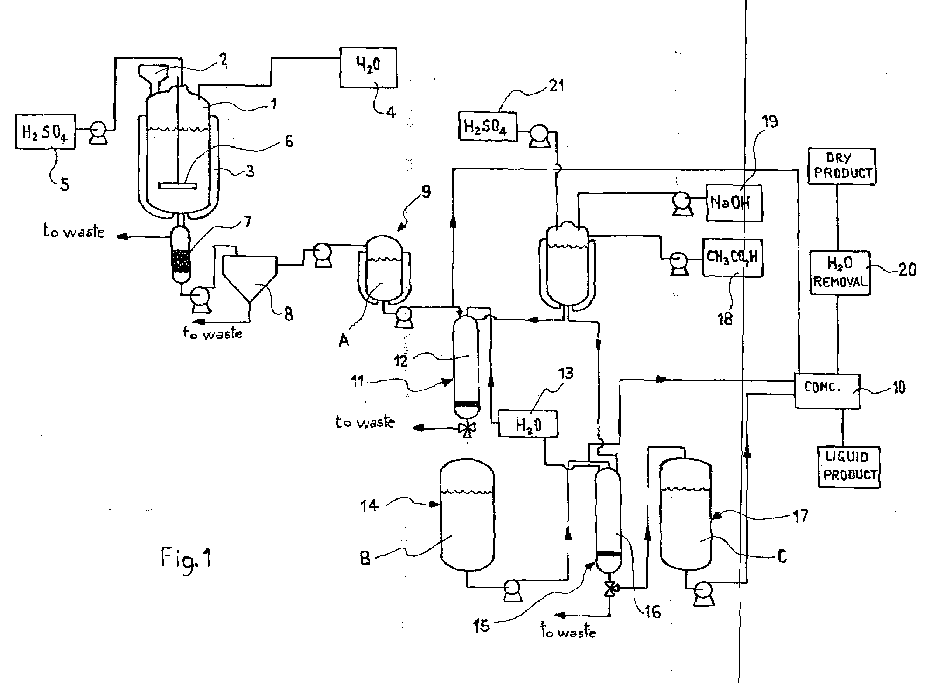

With reference to fig. 1, the invention apparatus comprises a means, or reactor, 1, preferably an autoclave, in which the hydrolysis according to the invention process is carried out. As previously mentioned, the hydrolysis is carried out in the autoclave 1 at a temperature within the range of 110 to 130°C, at a pressure of 10 to 20 psi and for a time length within the range of 15 to 45 minutes. Preferably, the hydrolysis temperature is within the range of 118 to 126 °C and in the most preferred embodiment the hydrolysis is carried out at 120-121 °C, at 15 psi for 30 minutes.

The autoclave 1 is provided with feeding means 2 for feeding to it the starting materials, namely the residues, or by-products, of olive oil extraction, namely pomaces (i.e. the solid residues of the pressed olives) or vegetation water (i.e. the water that separates from the oil in the process involving decanting or centrifugation with added water).

Autoclave 1 is preferably of the continuous type, i.e. it is suitable to treat the starting materials in a continuous process rather than batchwise. Heating means 3 for heating the reactor 1 to the above mentioned temperatures are provided in a known way, e.g. as a jacket around the reactor. Reactor 1 is also provided with a supply of demineralised water 4 and with means 6 to stir the water mixture of starting product. Demineralised water is added only if needed (e.g. no water is added when the starting material is vegetation water); usually, the ratio water to solids is within the range of 1:1 to 4:1

The hydrolysis process is carried out at a pH of 1.0 to 6.0, preferably of 1.0 to 3.0. The required amount of acid, preferably sulphuric acid, is obtained from acid tank 5.

The combination of acidic conditions and of temperature in the claimed ranges results in a hydrolysis process that is fast and efficient and can last for 30 minutes only, at 121°C and 15 psi. Moreover, the process also results in the sterilization of the hydrolysis products.

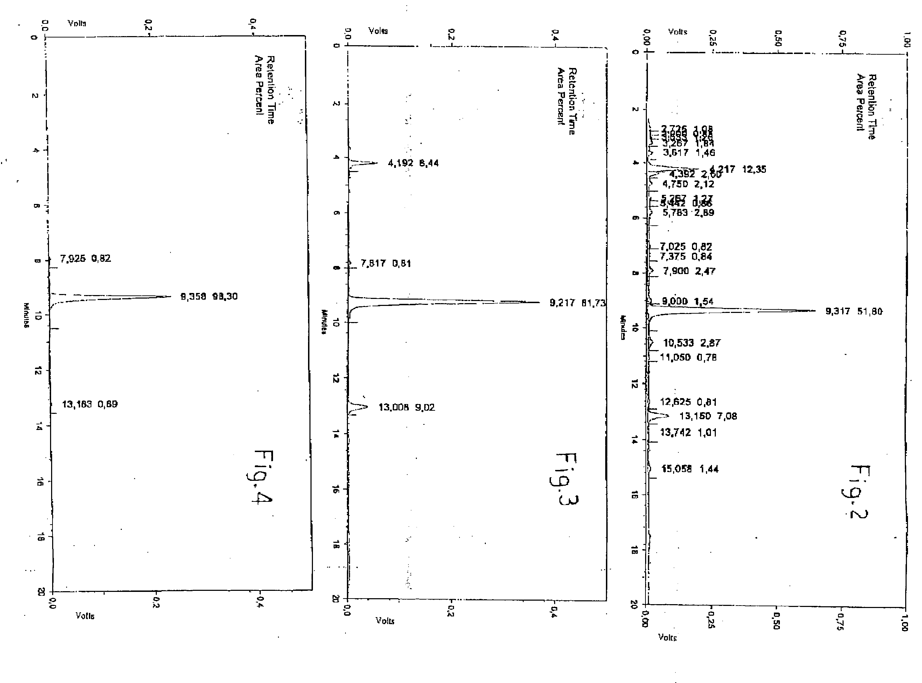

The outlet of autoclave, i.e. reactor, 1 is connected with a filter 7 for removing solids from the reaction mixture containing hydroxytyrosol and other phenols. The filtered portion is then sent to a further separation means, preferably a centrifuge 8, where further solids are removed from the reaction mixture to obtain a liquid substantially free from solids and suitable for the following purification or/and concentration steps. The clarified liquid after centrifugation has a brownish colour and is preferably stored in reservoir 9. The clarified liquid A is now containing hydroxytyrosol, a residue of oleuropeine and minor amounts of phenols and other products. Fig. 2 chromatogram shows the composition and the percent of the detected compounds in this liquid and is obtained by HPLC at 280 n,m; the major peak at 9.317 is hydroxytyrosol and the peak at 13.150 is tyrosol.

According to a preferred embodiment of the invention, liquid A is concentrated by evaporation or tangential flow filtration (TFF), preferably by using a reverse osmosis system. The concentration step can be carried out on liquid A directly or after a purification step by means of at least one chromatographic column. To this end, storage tank, or reservoir, 9 is connected with means of concentrating 10, e.g. by TFF or preferably by reverse osmosis, and to purification means.

Purification means comprises at least one chromatographic column of a resin selected from acid activated anion exchange resins, and adsorbent non-ionic resins; in a preferred embodiment, the acid activated anion exchange resin is a weakly basic, water elutable anion exchange resin, and the adsorbent non-ionic resin is a macroreticular cross-linked aromatic polymer.

The anion exchange resins used as a chromatographic resin for the purification of hydroxytyrosol of the present invention are not particularly limited so long as they can be acid activated. Examples of preferred weakly basic anion exchange resins include polyamine-type resins (including polyamine-type chelating resins) such as reaction products of a styrene/divinylbenzene copolymer and diethylenetriamine or other, and resins as polymerization products of compounds mainly comprising allylamine, vinylamine or other; and acrylic resins such as copolymers of divinylbenzene and amide compounds which comprise acrylic acid or methacrylic acid and dimethylaminopropylamine or other. Other resins may also be used in which the aforementioned weakly basic anion exchange resin is partly substituted with strongly basic exchange groups such as trimethylamine, dimethylethanolamine or other. More specifically, known in the art resins which have been used so far can be used, for example, Diaion WA10, WA20, WA21 and WA30 (Mitsubishi Chemical), Amberlite IRA-35, IRA-67 (IRA-68), IRA-93ZU, IRA-94S, IRA-478 (Rhöm & Haas), WGR-2 (Dow Chemical) and other.

According to the invention process, these resins are acid activated before being used, preferably with acetic acid. These resins are particularly suitable for the invention process in view of their low cost and of the fact that they can be regenerated in mild conditions. Additionally, the weakly basic nature of the resin allows the partial separation of hydroxytyrosol and tyrosol molecules. From the comparison of the peak ratio hydroxytyrosol/tyrosol and the HPLC purity of the hydroxytyrosol peak of Figs.2 and 3, it can be appreciated the efficiency of the chromatographic separation according to the invention. In fact, the use of a weakly anionic exchange resin allows to separate very similar compounds during the elution, this is not possible by the prior art techniques using strong anionic exchange resins, because the elution of such resins is generally an all-or-nottiing process. As previously mentioned, a further advantage is that the retained products can be eluted with water, without using any other polar solvent such as methanol.

Suitable adsorption resins are based on non-ionic, hydrophobic, macroreticular cross-linked aromatic polymer. Such resins are typically aromatic-polymers, such as styrene and divinylbenzene copolymers, which may be cross-linked. Such resins are known and are generally prepared by polymerization of the appropriate monomers. The adsorption resins used as a chromatographic resin for the purification of hydroxytyrosol of the present invention are not particularly limited so long as they can be water eluted. Examples of preferred adsorption resins include: Amberlite. RTM. XAD-4, XAD-7, XAD-1180, XAD-16 and XAD-1600 (available from Rohm & Haas); XUS-40323.00, XUS-40285.00 and XUS-40283.00 (available from Dow Chemical Co.); and SP-700, SP-825, SP850, Diaion HP 10, HP 20, HP 30, HP 40 and HP 50 (available from Mitsubishi Chemical).

These type of resins are particularly suitable for the invention process in view of their very high adsorption capacity for hydroxytyrosol and of the fact that the adsorbed hydroxytyrosol can be recovered by elution with water, only, without any polar solvent such as methanol or ethanol, as was instead required by the prior art techniques using different resins. Adsorbed hydroxytyrosol is recovered substantially quantitatively.

In the shown preferred embodiment, the process of the invention provides for a two-step purification on chromatographic columns.

Liquid A, as obtained from the initial steps a) and b), i.e. hydrolysis and solid separation, is charged into column 11, containing the anion exchange resin 12 as above detailed. The permeate is sent to waste treatment (not shown). Demineralized water from water supply 13 is then fed to column 11 to elute the retained products and the eluted liquid is collected in reservoir 14. The thus obtained liquid product (liquid B) has a purity in hydroxytyrosol of at least 75%, and generally of at least 80%, the purity being determined as the % of the peak areas in a chromatogram by HPLC at 280 nm. The recovery of retained hydroxytyrosol from the resin is at least 85% and is generally at least 90%.

Figure 3 shows the relevant HPLC chromatogram of the obtained purified liquid product B.

In the second purification step, liquid product B is charged into column 15. containing a non-ionic adsorption resin 16, as above detailed. The permeate is sent to waste treatment and the adsorbed hydroxytyrosol is recovered by elution with demineralized water from supply 13 and is collected in reservoir 17.

Liquid C, i.e. the liquid collected in reservoir 17 has a purity in hydroxytyrosol of at least 90%, and generally of at least 95%, the purity being determined as the % of the peak areas in a chromatogram by HPLC at 280 nm. The recovery of retained hydroxytyrosol from the resin is at least 90% and is generally at least 95% and substantially quantitative. Figure 4 shows the relevant HPLC chromatogram of the obtained purified liquid product C.

Fig. 1 also shows a source of acetic acid 18 that is connected to column 11 for acid activation of resin 12 and a source of NaOH 19 or other suitable base for regeneration of the same. Additionally, a source of NaOH 19 for regeneration of resin 16 is connected to column 15 and a source of sulphuric acid 21 for resin surface activation is also shown.

As for liquid A, also liquid B and liquid C can be concentrated in concentration means 10, e.g. by evaporation or TFF, preferably by reverse osmosis, to a hydroxytyrosol content that can reach 40%.

In a further step of the invention process, the liquid products obtained by the previously discussed steps are dried in dryer means 20, e.g. freeze-dryer, vacuum rotoevaporator or preferably by spray-dryer, to produce a solid final product. The final product characteristics will be different according to the starting product (A, B or C) that is dried, the purity of the dry product being within the range of 45% to 99% (HPLC at 280 nm).

This drying step is preferably carried out on liquid products B and C, after concentrating them as above disclosed. The drying step can advantageously make use of carriers suitable for the final use of the dry product; suitable carriers are e.g. maltodextrines, lactose, caseinates etcetera.

Suitable drying techniques are known in the art and comprise spray drying (usually with the use of carriers), freeze-drying and water evaporation under vacuum. The resulting products will have a hydroxytyrosol content of 0.5% to 5.0% (w/w) if a carrier is used, the hydroxytyrosol content can reach up to about 95% (w/w) if no carrier is used. It should be noticed that the invention process provides a purified liquid product (B and especially C) that is so pure that it can be evaporated to a dry powder even without carriers, this being not possible with known techniques.

The invention will now be further disclosed with reference to the following non-limiting examples.

### EXAMPLE 1

### hydroxytyrosol extraction from olive waste (alperujo), purification of the water phase

250 g of a sample of dry olive waste are mixed with 838 ml of demineralized water and 16.7 g of sulphuric acid (98%). The obtained mixture is kept in autoclave for 30 minutes at 121 °C. After that, the aqueous phase is separated from the solid residue, by filtering on a filter. The solid phase, retained on the filter, is washed with 310 ml of demineralized water, and the water coming from this washing operation is collected with the aqueous phase previously recovered. The aqueous phase, approximately 835 ml, is concentrated by evaporation in order to reach a final volume of about 193 ml. The aqueous phase is then centrifuge refined to eliminate solid particles passed through the filter. After solid elimination, 160 ml of crude aqueous extract, containing 1.41 g of hydroxytyrosol, with a HPLC purity of 47.5%, are obtained.

### EXAMPLE 2

### Ion exchange hydroxytyrosol purification

A sample of 160 ml of crude aqueous extract containing 1.41 g of hydroxytyrosol obtained according to Example 1, is loaded on a column containing a ion exchange resin of the anionic type, previously activated by means of acetate cycle. For example, Diaion WA10 may be used. The liquid phase recovered at the end of the Column, does not contain any hydroxytyrosol, which is instead continuously eluted with demineralised, water, until at least 90% of the initially charged hydroxytyrosol is recovered. The eluted phase contains approximately 1.27 g of hydroxytyrosol with an HPLC purity of about 80.85%.

### EXAMPLE 3

### Ion exchange and adsorption hydroxytyrosol purification

A sample of 160 ml of crude aqueous extract containing 1.41 g of hydroxytyrosol obtained according to Example 1, is loaded on a column containing an ion exchange resin of the anionic type, previously activated by means of acetate cycle. For example, IRA-67 may be used. The liquid phase recovered at the end of the column, does not contain any hydroxytyrosol, which is instead continuously eluted with demineralised water until at least 90% of the initially charged hydroxytyrosol is recovered.

The eluted phase coming from the first column, is charged on a column containing an adsorption resin. For example, resin XAD-1180 may be used. The liquid phase recovered at the end of the column, does not contain any hydroxytyrosol. Then, hydroxytyrosol is eluted from the resin with demineralised water until at least 90% of the initially charged hydroxytyrosol is recovered.

The eluted phase contains approximately 1.14 g of hydroxytyrosol with an HPLC purity of about 95.72%.

### EXAMPLE 4

### Concentration of hydroxytyrosol crude extract enriched by reverse osmosis

A sample of 15.8 I of crude aqueous extract containing 137 g of hydroxytyrosol obtained in a pilot plant according to Example 1 (but excluding concentration by evaporation), is concentrated using a reverse osmosis pilot plant equipped with a polymeric membrane, in order to obtain an olive extract concentrate containing 11.25% of hydroxytyrosol with an HPLC purity of 46.95%.

### EXAMPLE 5

### Concentration of hydroxytyrosol ion exchange purified extract enriched by reverse osmosis

A sample of 80 l of crude aqueous extract containing 150 g of hydroxytyrosol obtained in a pilot plant according to Example 2, is concentrated using a reverse osmosis pilot plant, equipped with a 2.5 m² polymeric membrane, in order to reduce the volume to 10 1 of concentrate product. A 0.3 m² membrane made of the same material is then used in order to obtain an hydroxytyrosol concentrate containing 10.80% of hydroxytyrosol with an HPLC purity of 80.53%.

### EXAMPLE 6

### Concentration of hydroxytyrosol ion exchange and adsorption purified extract by reverse osmosis

A sample of 546 l of crude aqueous extract containing 135 g of hydroxytyrosol obtained in a pilot plant according to Example 3, is concentrated using a reverse osmosis pilot plant, equipped with a 2.5 m² polymeric membrane, in order to reduce the volume to 10 l of concentrate product. A 0.3 m² membrane made of the same material is then used, in order to obtain an hydroxytyrosol concentrate containing 12.20% of hydroxytyrosol with an HPLC purity of 95.27%.

### EXAMPLE 7

### Spray-drying of the crude extract enriched in hydroxytyrosol without any purification

A sample of 92 ml of crude aqueous extract containing 1.02 g of hydroxytyrosol obtained according to Example 1, is mixed with 350 ml of demineralised water and 100 g of maltodextrin. For example, equivalent 10 dextrose potato maltodextrin may be used. A peristaltic pump is used to feed the spray-dryer, which is previously equilibrated with an inlet air temperature of 150°C. The feeding speed is adjusted in order to obtain an outlet air temperature which is less than 100°C, 95 g of a brown powder, with a moisture of 6.85% (Karl Fischer) and a hydroxytyrosol richness of 0.98%, are obtained.

### EXAMPLE 8

### Spray-drying of the partially purified aqueous extract enriched in hydroxytyrosol

A sample of 29 ml of aqueous extract containing 0.38 g of hydroxytyrosol obtained according to Example 2 and subsequently concentrated by reverse osmosis until 13.19 g/l, is mixed with 150 ml of demineralised water and 50 g of maltodextrin. For example, equivalent 10 dextrose potato maltodextrin may be used. A peristaltic pump is used to feed the spray-dryer, which is previously equilibrated with an inlet air temperature of 150°C. The feeding speed is adjusted in order to obtain an outlet air temperature which is less than 100°C. 48.25 g of a greyish powder, with a moisture of 6.72% (Karl Fischer) and a hydroxytyrosol richness of 0.71 %, are obtained.

### EXAMPLE 9

### Spray-drying of the purified aqueous extract enriched in hydroxytyrosol

A sample of 188 ml of purified aqueous extract containing 0.29 g of hydroxytyrosol obtained according to Example 3, and subsequently concentrated by reverse osmosis, is slowly stirred with 28.5 g of maltodextrin. For example, equivalent 10 dextrose potato maltodextrin may be used. A peristaltic pump is used to feed the spray-dryer, which is previously equilibrated with an inlet air temperature of 175°C. The feeding speed is adjusted in order to obtain an outlet air temperature which is less than 100°C. 27.1 g of a white powder, with a moisture of 5.45% (Karl Fischer) and a hydroxytyrosol richness of 0.97%, are obtained.

### EXAMPLE 10

### Preparation of a highly rich hydroxytyrosol powder

A sample of 1750 I of aqueous extract containing 432 g of hydroxytyrosol obtained, according to Example 3, in a pilot plant is concentrated according to Example 6, to obtain an hydroxytyrosol concentrate containing 39.04% of hydroxytyrosol with an HPLC purity of 95.60%. The concentrated solution is used to feed the spray-dryer, which is previously equilibrated with an inlet air temperature of 150°C. The feeding speed is adjusted in order to obtain an outlet air temperature less than 100°C. 375.84 g of a light brown powder, with a moisture of 4.35% (Karl Fischer) and a hydroxytyrosol richness of 9,4.74%, are obtained.

### EXAMPLE 11

### Hydroxytyrosol extraction from three phases pomaces (orujo), purification of the aqueous phase

475.5 g of a sample of three phases pomace with a humidity of 60.55%, are mixed with 800 ml of demineralized water and 26.36 g of sulphuric acid (98%). The obtained mixture is kept in autoclave for 30 minutes at 121 °C. After that, the aqueous phase is separated from the solid residue, by filtering on a 600 micron polypropylene filter. The filtered aqueous phase, approximately 795 ml; is concentrated by evaporation in order to reach a final volume of about 343.8 ml.

The aqueous phase is then centrifuge refined to eliminate solid particles passed through the filter. After solids elimination, 275 ml of crude aqueous extract, containing 0.97 g of hydroxytyrosol, with a HPLC purity of 54%, are obtained.

### EXAMPLE 12

### Ion exchange and adsorption purification of hydroxytyrosol deriving from three phases pomace (orujo)

A sample of 275 ml of crude aqueous extract containing 0.97 g of hydroxytyrosol obtained according to Example 11, is loaded on a column containing an ion exchange resin of the anionic kind, previously activated by means of acetate cycle. For example, Diaion WA10 resin may be used. The liquid phase recovered at the end of the column, does not contain any hydroxytyrosol, which is instead continuously eluted with demineralised water until at least 90% of the initially charged hydroxytyrosol is recovered.

The eluted phase coming from the first column is loaded on a column containing an adsorption resin. For example, resin Diaion HP20 may be used. The liquid phase recovered at the end of the column, does not contain any hydroxytyrosol. Then, hydroxytyrosol is eluted from the resin with demineralised water until at least 90% of the initially charged hydroxytyrosol is recovered.

The eluted phase contains approximately 0.80 g of hydroxytyrosol with an HPLC purity which is higher than 95%.

## Claims

1. A process of producing hydroxytyrosol from a by-product of the olive oil extraction, **characterized in** comprising the step of:
a) carrying out said acid hydrolysis of said by-product at a temperature within the range of 105°C to 140°C and at a pH within the range of 1.0 to 6.0.

2. A process according to claim 1, wherein said hydrolysis is carried out in an autoclave and the hydrolysis temperature is within the range of 110°C to 130°C.

3. A process according to claim 1 or 2, wherein the duration of said hydrolysis step is within the range of 15 to 45 minutes.

4. A process according to any claim 1 to 3, further comprising the step of:
b) subsequently treating the hydrolysis product of step a) to obtain a clarified aqueous solution.

5. A process according to any previous claim, further comprising a step of reverse osmosis concentration.

6. A process according to any previous claim, further comprising the steps of:
c) loading the product obtained from step b) in at least one chromatographic column of a resin selected from acid activated anion exchange resins, and adsorbent non-ionic resins;
d) eluting the products retained in said chromatographic columns with water.

7. A process according to claim 6, comprising the steps of: loading the product obtained from step b) in a chromatographic column of an acid activated anion exchange resin, eluting the retained products with water, loading the eluted products in a chromatographic column of an adsorbent non-ionic resin, eluting the retained product from said column with water.

8. A process according to any previous claim, further comprising the step of water removal until the product is in a solid form. :

9. A hydroxytyrosol containing product as obtainable through a process according to any of the previous claims, and having a hydroxytyrosol content of at least 0.5% (w/w) and a purity of at least 40% (by HPLC 280nm).

10. A hydroxytyrosol containing product, as obtainable through a process according to any claim 5 to 7, having a hydroxytyrosol content of at least 2% (w/w) and a purity of at least 80% (by HPLC 280nm).

11. A hydroxytyrosol containing solid product, having a hydroxytyrosol content of at least 0.5% (w/w) and a purity of at least 40% (by HPLC 280nm) and a total phenol content of at least 1 %.

12. A hydroxytyrosol containing solid product according to claim 11, having a hydroxytyrosol content of at least 90% (w/w) and a purity of at least 90% (by HPLC 280nm) and a total phenols content of at least 92%.

13. An apparatus for producing a hydroxytyrosol containing product from a by-product of the olive oil extraction comprising means of acid hydrolysis of said by-product, **characterized in** comprising means for carrying out said acid hydrolysis at a temperature within the range of 105°C to 140°C and at a pH within the range of 1.0 to 6.0; and means for treating the hydrolysis product to obtain a clarified aqueous solution

14. An apparatus according to claim 13, wherein said means for acid hydrolysis comprise an autoclave for the treatment of a continuous flow of by-products.

15. An apparatus according to claim 13 or 14, further comprising at least one chromatographic column of a resin selected from an acid activated anion exchange resins that is a weakly basic, water elutable anion exchange resin, and an adsorbent non-ionic resin that is a macroreticular cross-linked aromatic polymer.

16. An apparatus according to any claim 13 to 15, further comprising reverse osmosis means.

17. An apparatus according to any claim 13 to 16, further comprising water removal means.

18. An apparatus according to claim 17, wherein said means for water removal comprise a spray-dryer system.
